Europäisches Patentamt

European Patent Office

Office européen des brevets

(11) Publication number: **0 331 514**
**A2**

(12) **EUROPEAN PATENT APPLICATION**

(21) Application number: **89302145.1**

(22) Date of filing: **03.03.89**

(51) Int. Cl.⁴: **G 01 N 33/68**
**C 12 P 21/00, C 07 K 13/00,**
**C 07 K 7/00**

(30) Priority: **04.03.88 JP 51313/88**
**21.09.88 JP 237200/88**

(43) Date of publication of application:
**06.09.89 Bulletin 89/36**

(84) Designated Contracting States: **DE FR GB**

(71) Applicant: **AJINOMOTO CO., INC.**
**5-8, Kyobashi 1-chome, Chuo-ku**
**Tokyo 104 (JP)**

(72) Inventor: **Ishiguro, Tsuneo c/o Central Research Lab**
**Ajinomoto Co Inc No 1-1 Suzuki-cho**
**Kawasaki-ku Kawasaki-shi Kanagawa-ken (JP)**

**Eguchi Chikahiko c/o Central Research Lab**
**Ajinomoto Co Inc No 1-1 Suzuki-cho**
**Kawasaki-ku Kawasaki-shi Kanagawa-ken (JP)**

(74) Representative: **Armitage, Ian Michael et al**
**MEWBURN ELLIS & CO. 2/3 Cursitor Street**
**London EC4A 1BQ (GB)**

(54) Assaying the duchenne muscular dystrophy protein deletion or defect.

㊏ This invention provides a method for assaying for the protein deletion or defect associated with the human DMD gene, which comprises preparing a peptide which contains at least part of an amino acid sequence encoded by a Duchenne muscular dystrophy gene or a derivative thereof, administering said peptide or derivative thereof to a mammal to form antiserum, then combining said antiserum, or an antibody fraction separated from said antiserum, with a substance to be tested and assaying for any antigen-antibody complex formed.

It also provides methods for preparing the antiserum by use of part of the amino acid sequence encoded by the human Duchenne muscular dystrophy gene. The invention also provides kits and reagents for such assays.

**Description**

## ASSAYING THE DUCHENNE MUSCULAR DYSTROPHY PROTEIN DELETION OR DEFECT

The present invention relates to Duchenne muscular dystrophy. It provides an assay for the protein deletion or defect of the disease, reagents and kits for the assay, and means for making the reagents.

Duchenne muscular dystrophy (DMD) is a hereditary disease which develops almost exclusively in males. A gene located on the X chromosome which appears to be associated with the protein deletion or defect peculiar to this disease has been identified and sequenced. (M. König, E.P. Hoffman, C.J. Bertelson, A.P. Monaco, C. Feener and L.M. Kunkel: Cell 50, 509 (1987), E.P. Hoffman, A.P. Monaco, C.C. Feener and L.M. Kunkel, Science, 238, 347 (1987).

The production of antibodies capable of specifically recognizing a protein encoded by this gene would allow this protein to be assayed, thereby providing a diagnostic tool which may be useful in identifying the presence/absence of the protein deletion or defect and thereby of the hereditary disease. This has been attempted by Hoffman et al, using a protein encoded by an equivalent gene in mice suffering from a disease which is similar to Duchenne muscular dystrophy. (E.P. Hoffman, R.H. Brown, Jr. and L.M. Kunkel, Cell, 51, 919 (1987); E.P. Hoffman, C.M. Knudson, K.P. Campbell and L.M. Kunkel, Nature, 330, 754 (1987)). However, the antibodies produced by Hoffman et al have limited use in any diagnostic assay for the protein deletion or defect encoded by the human gene. Firstly, the amino acid sequence of the mouse protein used by Hoffman et al differs from the equivalent human protein by about 10%. Secondly, the protein used by Hoffman et al was one of high molecular weight (208 or 410 amino acid residues). When such high molecular weight proteins are used as antigens in methods for raising antibodies, antibodies are frequently raised to non-specific areas of the protein and therefore these antibodies may cross-react with proteins other than that protein used as the original antigen. Because of these two problems, the antibodies produced by Hoffman et al lack specificity and are therefore of little diagnostic value. Hoffman et al attempted to overcome the second of these two problems by using a specimen obtained by previously homogenizing cells to be tested followed by separating protein from the homogenate by electrophoresis and then performing an antigen-antibody reaction with respect to the specimen. However, such a procedure is complicated.

Thus the available methods for determining the DMD protein deletion or defect associated with the disease, have disadvantages as the antibodies available for use in any diagnostic methods lack specificity and the techniques to compensate the specificity of these antibodies are complicated.

The present invention provides a method for assaying the protein deletion or defect associated with the human DMD gene, which comprises preparing a peptide which contains at least part of an amino acid sequence encoded by a Duchenne muscular dystrophy gene or a derivative thereof, administering said peptide or derivative thereof to a mammal to form antiserum, then combining said antiserum, or an antibody fraction separated from said antiserum, with a substance to be tested and assaying any antigen-antibody complex formed.

The present invention additionally provides a method for assaying the protein deletion or defect associated with the human Duchenne muscular dystrophy gene, which comprises preparing a peptide which contains a part of an amino acid sequence encoded by the gene, or a derivative thereof, administering said peptide or a derivative thereof to a mammal, collecting spleen cells therefrom, making fused cells thereof, cloning said fused cells to form an antibody, then combining the antibody with a substance to be tested and assaying any antigen-antibody complex formed.

The present invention also provides methods for preparing an antiserum or an antibody fraction separated from the antiserum, capable of specifically reacting with the DMD protein, by use of at least part of the amino acid sequence encoded by the human DMD gene. Also provided are methods of purifying said antiserum and/or separating the antibody fraction therefrom, by use of at least part of the amino acid sequence encoded by the human DMD gene. The present invention also provides diagnostic assays which utilise the antiserum or antibody fraction thereof and kits for such assays. The invention also provides amino acid sequences encoded by the human DMD gene.

In the methods described above, the amino acid sequence encoded by the human DMD gene may be as shown in the following sequence 1:

```
MLWWEEVEDC   YEREDYQKKT   FTKWVNAQFS   KFGKQHIENL   FSDLQDGRRL        50
LDLLEGLTGQ   KLPKEKGSTR   VHALNNVVNKA  LRVLQNNNVD   LVNIGSTDIV        100
DGNHKLTLGL   IWNIILHWQV   KNVMKNIMAG   LQQTNSEKIL   LSWVRQSTRN        150
YPQVNVINFT   TSWSDGLALN   ALIHSHRPDL   FDWNSVVCQQ   SATQRLEHAF        200
NIARYQLGIE   KLLDPEDVDT   TYPDKKSILM   YITSLFQVLP   QQVSIEAIQE        250
VEMLPRPPKV   TKEEHFQLHH   QMHYSQQITV   SLAQGYERTS   SPKPRFKSYA        300
YTQAAYVTTS   DPTRSPFPSQ   HLEAPEDKSF   GSSLMESEVN   LDRYQTALEE        350
VLSWLLSAED   TLQAQGEISN   DVEVVKDQFH   THEGYMMDLT   AHQGRVGNIL        400
QLGSKLIGTG   KLSEDEETEV   QEQMNLLNSR   WECLRVASME   KQSNLHRVLM        450
DLQNQKLKEL   NDWLTKTEER   TRKMEEEPLG   PDLEDLKRQV   QQHKVLQEDL io     500
EQEQVRVNSL   THMVVVVDES   SGDHATAALE   EQLKVLGDRW   ANICRWTEDR        550
WVLLQDILLK   WQRLTEEQCL   FSAWLSEKED   AVNKIHTTGF   KDQNEMLSSL        600
QKLAVLKADL   EKKKQSMGKL   YSLKQDLLST   LKNKSVTQKT   EAWLDNFARC        650
WDNLVQKLEK   STAQISQAVT   TTQPSLTQTT   VMETVTTVTT   REQILVKHAQ        700
EELPPPPPQK   KRQITVDSEI   RKRLDVDITE   LHSWITRSEA   VLQSPEFAIF        750
RKEGNFSDLK   EKVNAIEREK   AEKFRKLQDA   SRSAQALVEQ   MVNEGVNADS        800
IKQASEQLNS   RWIEFCQLLS   ERLNWLEYQN   NIIAFYNQLQ   QLEQMTTTAE        850
NWLKIQPTTP   SEPTAIKSQL   KICKDEVNRL   SGLQPQIERL   KIQSIALKEK        900
GQGPMFLDAD   FVAFTNHFKQ   VFSDVQAREK   ELQTIFDTLP   PMRYQETMSA        950
IRTWVQQSET   KLSIPQLSVT   DYEIMEQRLG   ELQALQSSLQ   EQQSGLYYLS        1000
TTVKEMSKKA   PSEISRKYQS   EFEEIEGRWK   KLSSQLVEHC   QKLEEQMNKL        1050
RKIQNHIQTL   KKWMAEVDVF   LKEEWPALGD   SEILKKQLKQ   CRLLVSDIQT        1100
IQPSLNSVNE   GGQKIKNEAE   PEFASRLETE   LKELNTQWDH   MCQQVYARKE       1150
ALKGGLEKTV   SLQKDLSEMH   EWMTQAEEEY   LERDFEYKTP   DELQKAVEEM        1200
KRAKEEAQQK   EAKVKLLTES   VNSVIAQAPP   VAQEALKKEL   ETLTTNYQWL        1250
CTRLNGKCKT   LEEVVWACWHE  LLSYLEKANK  WLNEVEFKLK   TTENIPGGAE        1300
EISEVLDSLE   NLMRHSEDNP   NQIRILAQTL   TDGGVMDELI   NEELETFNSR        1350
WRELHEEAVR   RQKLLEQSIQ   SAQETEKSLH   LIQESLTFID   KQLAAYIADK        1400
VDAAQMPQEA   QKIQSDLTSH   EISLEEMKKH   NQGKEAAQRV   LSQIDVAQKK        1450
LQDVSMKFRL   FQKPANFELR   LQESKMILDE   VKMHLPALET   KSVEQEVYQS        1500
QLNHCVNLYK   SLSEVKSEVE   MVIKTGRQIV   QKKQTENPKE   LDERVTALKL        1550
HYNELGAKVT   ERKQQLEKCL   KLSRKMRKEM   NVLTEWLAAT   DMELTKRSAV        1600
EGMPSNLDSE   VAWGKATQKE   IEKQKVHLKS   ITEVGEALKT   VLGKKETLVE        1650
DKLSLLNSNW   IAVTSRAEEW   LNLLLEYQKH   METFDQNVDH   ITKWIIQADT        1700
LLDESEKKKP   QQKEDVLKRL   KAELNDIRPK   VDSTRDQAAN   LMANRGDHCR        1750
KLVEPQISEL   NHRFAAISHR   IKTGKASIPL   KELEQFNSDI   QKLLEPLEAE        1800
IQQGVNLKEE   DFNKDMNEDN   EGTYKELLQR   GDNLQQRITD   ERKREEIKIK        1850
QQLLQTKHNA   LKDLRSQRRK   KALEISHQWY   QYKRQADDLL   KCLDDIEKKL        1900
ASLPEPRDER   KIKEIDRELQ   KKKEELNAVR   RQAEGLSEDG   AAMAVEPTQI        1950
QLSKRWREIE   SKFAQFRRLN   FAQIHTVREE   TMMVMTEDMP   LEISYVPSTY        2000
LTEITHVSQA   LLEVEQLLNA   PDLCAKDFED   LFKQEESLKN   IKDSLQQSSG        2050
RIDIIHSKKT   AALQSATPVE   RVKLQEALSQ   LDFQWEKVNK   MYKDRQGRFD        2100
RSVEKWRRFH   YDIKIFNQWL   TEAEQFLRKT   QIPENWEHAK   YKWYLKELQD        2150
GIGQRQTVVR   TLNATGEEII   QQSSKTDASI   LQEKLGSLNL   RWQEVCKQLS        2200
DRKKRLEEQK   NILSEFQRDL   NEFVLWLEEA   DNIASIPLEP   GKEQQLKEKL        2250
EQVKLLVEEL   PLRQGILKQL   NETGGPVLVS   APISPEEQDK   LENKLKQTNL        2300
QWIKVSRALP   EKQGEIEAQI   KDLGQLEKKL   EDLEEQLNHL   LLWLSPIRNQ        2350
LEIYNQPNQE   GPFDVQETEI   AVQAKQPDVE   EILSKGQHLY   KEKPATPVK        2400
RKLEDLSSEW   KAVNRLLQEL   RAKQPDLAPG   LTTIGASPTQ   TVTLVTQPVV        2450
TKETAISKLE   MPSSLMLEVP   ALADFNRAWT   ELTDWLSLLD   QVIKSQRVMV        2500
GDLEDINEMI   IKQKATMQDL   EQRRPQLEEL   ITAAQNLKNK   TSNQEARTII       2550
TDRIERIQNQ   WDEVQEHLQN   RRQQLNEMLK   DSTQWLEAKE   EAEQVLGQAR        2600
AKLESWKEGP   YTVDAIQKKI   TETKQLAKDL   RQWQTNVDYA   NDLALKLLRD        2650
```

```
YSADDTRKVH  MITENINASW  RSIHKRVSER  EAALEETHRL  LQQFPLDLEK   2700
FLAWLTEAET  TANVLQDATR  KERLLEDSKG  VKELMKQWQD  LQGEIEAHTD   2750
VYHNLDENSQ  KILRSLEGSD  DAVLLQRRLD  NMNFKWSELR  KKSLNIRSHL   2800
EASSDQWKRL  HLSLQELLVW  LQLKDDELSR  QAPIGGDFPA  VQKQNDVHRA   2850
FKRELKTKEP  VIMSTLETVR  IFLTEQPLEG  LEKLYQEPRE  LPPEERAQNV   2900
TRLLRKQAEE  VNTEWEKLNL  HSADWQRKID  ETLERLQELQ  EATDELDLKL   2950
RQAEVIKGSW  QPVGDLLIDS  LQDHLEKVKA  LRGEIAPLKE  NVSHVNDLAR   3000
QLTTLGIQLS  PYNLSTLEDL  NTRWKLLQVA  VEDRVRQLHE  AHRDFGPASQ   3050
HFLSTSYQGP  WERAISPNKV  PYYINHETQT  TCWDHPKMTE  LYQSLADLNN   3100
VRFSAYRTAM  KLRRLQKALC  LDLLSLSAAC  DALDQHNLKQ  NDQPMDILQI   3150
INCLTTIYDR  LEQEHNNLVN  VPLCVDMCLN  WLLNVYDTGR  TGRIRVLSFK   3200
TGIISLCKAH  LEDKYRYLFK  QVASSTGFCD  QRRLGLLLHD  SIQIPRQLGE   3250
VASFGGSNIE  PSVRSCFQFA  NNKPEIEAAL  FLDWMRLEPQ  SMVWLPVLHR   3300
VAAAETAKHQ  AKCNICKECP  IIGFRYRSLK  HFNYDICQSC  FFSGRVAKGH   3350
KMHYPMVEYC  TPTTSGEDVR  DFAKVLKNKF  RTKRYFAKHP  RMGYLPVQTV   3400
LEGDNMETPV  TLINFWPVDS  APASSPQLSH  DDTHSRIEHY  ASRLAEMENS   3450
NGSYLNDSIS  PNESIDDEHL  LIQHYCQSLN  QDSPLSQPRS  PAQILISLES   3500
EERGELERIL  ADLEEENRNL  QAEYDRLKQQ  HEHKGLSPLP  SPPEMMPTSP   3550
QSPRDAELIA  EAKLLRQHKG  RLEARMQILE  DHNKQLESQL  HRLRQLLEQP   3600
QAEAKVNGTT  VSSPSTSLQR  SDSSQPMLLR  VVGSQTSDSM  GEEDLLSPPQ   3650
DTSTGLEEVM  EQLNNSFPSS  RGRNTPGKPM  REDTM
```

In the method described above the peptide containing a part of the amino acid sequence encoded by the DMD gene may be a peptide containing a part of the amino acid sequence 1 shown by, for example:

a sequence of at least 10 amino acids from the sequence encoded by amino acids 1 to 3685 (inclusive);

a sequence of 10 to 200 amino acids from the sequence encoded by amino acid 1 to 3000 (inclusive);

a sequence of 10 to 200 amino acids from the sequence encoded by amino acids 1 to 2000 (inclusive);

a sequence of 10 to 200 amino acids from the sequence encoded by amino acids 1 to 1000 (inclusive);

a sequence of 10 to 200 amino acids from the sequence encoded by amino acids 1 to 500 (inclusive);

a sequence of 10 to 200 amino acids from the sequence encoded by amino acids 151 to 350 (inclusive);

a sequence of 10 to 100 amino acids from the sequence encoded by amino acids 1 to 3685 (inclusive);

a sequence of 10 to 100 amino acids from the sequence encoded by amino acids 1 to 3000 (inclusive);

a sequence of 10 to 100 amino acids from the sequence encoded by amino acids 1 to 2000 (inclusive);

a sequence of 10 to 100 amino acids from the sequence encoded by amino acids 1 to 1000 (inclusive);

a sequence of 10 to 100 amino acids from the sequence encoded by amino acids 1 to 500 (inclusive);

a sequence of 10 to 100 amino acids from the sequence encoded by amino acids 191 to 290 (inclusive);

a sequence of 10 to 60 amino acids from the sequence encoded by amino acids 1 to 3685 (inclusive);

a sequence of 10 to 60 amino acids from the sequence encoded by amino acids 1 to 3000 (inclusive);

a sequence of 10 to 60 amino acids from the sequence encoded by amino acids 1 to 2000 (inclusive);

a sequence of 10 to 60 amino acids from the sequence encoded by amino acids 1 to 1000 (inclusive);

a sequence of 10 to 60 amino acids from the sequence encoded by amino acids 1 to 500 (inclusive);

a sequence of 10 to 60 amino acids from the sequence encoded by amino acids 151 to 350 (inclusive);

a sequence of 10 to 60 amino acids from the sequence encoded by amino acids 190 to 290 (inclusive);

a sequence of 10 to 50 amino acids from the sequence encoded by amino acids 215 to 264 (inclusive);

a sequence of 20 to 50 amino acids from the sequence encoded by amino acids 215 to 264 (inclusive);

a sequence of 30 to 50 amino acids from the sequence encoded by amino acids 215 to 264 (inclusive);

a sequence of 40 to 50 amino acids from the sequence encoded by amino acids 215 to 264 (inclusive);

a sequence of 50 amino acids from the sequence encoded by amino acids 215 to 264 (inclusive);

a sequence of 10 to 200 amino acids from the sequence encoded by amino acids 201 to 600 (inclusive);

a sequence of 10 to 200 amino acids from the sequence encoded by amino acids 391 to 590 (inclusive);

a sequence of 10 to 100 amino acids from the sequence encoded by amino acids 201 to 600 (inclusive);

a sequence of 10 to 100 amino acids from the sequence encoded by amino acids 301 to 550 (inclusive);

a sequence of 10 to 100 amino acids from the sequence encoded by amino acids 416 to 515 (inclusive);

a sequence of 10 to 60 amino acids from the sequence encoded by amino acids 201 to 600 (inclusive);

a sequence of 10 to 60 amino acids from the sequence encoded by amino acids 301 to 550 (inclusive);

a sequence of 10 to 60 amino acids from the sequence encoded by amino acids 416 to 515 (inclusive);

a sequence of 10 to 60 amino acids from the sequence encoded by amino acids 436 to 495 (inclusive);

a sequence of 10 to 50 amino acids from the sequence encoded by amino acids 440 to 489 (inclusive);
a sequence of 20 to 50 amino acids from the sequence encoded by amino acids 440 to 489 (inclusive);
a sequence of 30 to 50 amino acids from the sequence encoded by amino acids 440 to 489 (inclusive);
a sequence of 40 to 50 amino acids from the sequence encoded by amino acids 440 to 489 (inclusive);
a sequence of 50 amino acids from the sequence encoded by amino acids 440 to 489 (inclusive);
a sequence of 10 to 200 amino acids from the sequence encoded by amino acids 1001 to 3000 (inclusive);
a sequence of 10 to 200 amino acids from the sequence encoded by amino acids 2001 to 3000 (inclusive);
a sequence of 10 to 200 amino acids from the sequence encoded by amino acids 2101 to 2600 (inclusive);
a sequence of 10 to 200 amino acids from the sequence encoded by amino acids 2286 to 2485 (inclusive);
a sequence of 10 to 100 amino acids from the sequence encoded by amino acids 1001 to 3000 (inclusive);
a sequence of 10 to 100 amino acids from the sequence encoded by amino acids 2001 to 3000 (inclusive);
a sequence of 10 to 100 amino acids from the sequence encoded by amino acids 2101 to 2600 (inclusive);
a sequence of 10 to 100 amino acids from the sequence encoded by amino acids 2335 to 2434 (inclusive);
a sequence of 10 to 60 amino acids from the sequence encoded by amino acids 1001 to 3000 (inclusive);
a sequence of 10 to 60 amino acids from the sequence encoded by amino acids 2001 to 3000 (inclusive);
a sequence of 10 to 60 amino acids from the sequence encoded by amino acids 2101 to 2600 (inclusive);
a sequence of 10 to 60 amino acids from the sequence encoded by amino acids 2356 to 2415 (inclusive);
a sequence of 10 to 50 amino acids from the sequence encoded by amino acids 2360 to 2409 (inclusive);
a sequence of 20 to 50 amino acids from the sequence encoded by amino acids 2360 to 2409 (inclusive);
a sequence of 30 to 50 amino acids from the sequence encoded by amino acids 2360 to 2409 (inclusive);
a sequence of 40 to 50 amino acids from the sequence encoded by amino acids 2360 to 2409 (inclusive);
a sequence of 50 amino acids from the sequence encoded by amino acids 2360 to 2409 (inclusive);
a sequence of 10 to 200 amino acids from the sequence encoded by amino acids 1001 to 3685 (inclusive);
a sequence of 10 to 200 amino acids from the sequence encoded by amino acids 2001 to 3685 (inclusive);
a sequence of 10 to 200 amino acids from the sequence encoded by amino acids 3001 to 3685 (inclusive);
a sequence of 10 to 200 amino acids from the sequence encoded by amino acids 3401 to 3600 (inclusive);
a sequence of 10 to 100 amino acids from the sequence encoded by amino acids 1001 to 3685 (inclusive);
a sequence of 10 to 100 amino acids from the sequence encoded by amino acids 2001 to 3685 (inclusive);
a sequence of 10 to 100 amino acids from the sequence encoded by amino acids 3001 to 3685 (inclusive);
a sequence of 10 to 100 amino acids from the sequence encoded by amino acids 3471 to 3570 (inclusive);
a sequence of 10 to 60 amino acids from the sequence encoded by amino acids 1001 to 3685 (inclusive);
a sequence of 10 to 60 amino acids from the sequence encoded by amino acids 2001 to 3685 (inclusive);
a sequence of 10 to 60 amino acids from the sequence encoded by amino acids 3001 to 3685 (inclusive);
a sequence of 10 to 60 amino acids from the sequence encoded by amino acids 3401 to 3600 (inclusive);
a sequence of 10 to 60 amino acids from the sequence encoded by amino acids 3470 to 3569 (inclusive);
a sequence of 10 to 50 amino acids from the sequence encoded by amino acids 3495 to 3544 (inclusive);
a sequence of 20 to 50 amino acids from the sequence encoded by amino acids 3495 to 3544 (inclusive);
a sequence of 30 to 50 amino acids from the sequence encoded by amino acids 3495 to 3544 (inclusive);
a sequence of 40 to 50 amino acids from the sequence encoded by amino acids 3495 to 3544 (inclusive);
a sequence of 50 amino acids from the sequence encoded by amino acids 3495 to 3544 (inclusive);
a sequence of 10 to 200 amino acids from the sequence encoded by amino acids 1 to 200 (inclusive);
a sequence of 10 to 100 amino acids from the sequence encoded by amino acids 1 to 200 (inclusive);
a sequence of 10 to 100 amino acids from the sequence encoded by amino acids 1 to 100 (inclusive);
a sequence of 10 to 60 amino acids from the sequence encoded by amino acids 1 to 100 (inclusive);
a sequence of 10 to 60 amino acids from the sequence encoded by amino acids 6 to 65 (inclusive);
a sequence of 10 to 50 amino acids from the sequence encoded by amino acids 11 to 60 (inclusive);
a sequence of 20 to 50 amino acids from the sequence encoded by amino acids 11 to 60 (inclusive);
a sequence of 30 to 50 amino acids from the sequence encoded by amino acids 11 to 60 (inclusive);
a sequence of 40 to 50 amino acids from the sequence encoded by amino acids 11 to 60 (inclusive);
a sequence of 50 amino acids from the sequence encoded by amino acids 11 to 60 (inclusive);
in the aforesaid amino acid sequence 1.

In the methods described above, the amino acid sequence encoded by the human DMD gene may be as shown in the following sequence 2:

EP 0 331 514 A2

```
MLWWEEVEDC YEREDVQKKT FTKWVNAQFS KFGKQHIENL FSDLQDGRRL      50
LDLLEGLTGQ KLPKEKGSTR VHALNNVNKA LRVLQNNNVD LVNIGSTDIV     100
DGNHKLTLGL IWNIILHWQV KNVMKNIMAG LQQTNSEKIL LSWVRQSTRN     150
YPQVNVINFT TSWSDGLALN ALIHSHRPDL FDWNSVVCQQ SATQRLEHAF     200
NIARYQLGIE KLLDPEDVDT TYPDKKSILM YITSLFQVLP QQVSIEAIQE     250
VEMLPRPPKV TKEEHFQLHH QMHYSQQITV SLAQGYERTS SPKPRFKSYA     300
YTQAAYVTTS DPTRSPFPSQ HLEAPEDKSF GSSLMESEVN LDRYQTALEE     350
VLSWLLSAED TLQAQGEISN DVEVVKDQFH THEGYMMDLT AHQGRVGNIL     400
QLGSKLIGTG KLSEDEETEV QEQMNLLNSR WECLRVASME KQSNLHRVLM     450
DLQNQKLKEL NDWLTKTEER TRKMEEEPLG PDLEDLKRQV QQHKVLQEDL     500
EQEQVRVNSL THMVVVVDES SGDHATAALE EQLKVLGDRW ANICRWTEDR     550
WVLLQDILLK WQRLTEEQCL FSAWLSEKED AVNKIHTTGF KDQNEMLSSL     600
QKLAVLKADL EKKKQSMGKL YSLKQDLLST LKNKSVTQKT EAWLDNFARC     650
WDNLVQKLEK STAQISQAVT TTQPSLTQTT VMETVTTVTT REQILVKHAQ     700
EELPPPPPQK KRQITVDSEI RKRLDVDITE LHSWITRSEA VLQSPEFAIF     750
RKEGNFSDLK EKVNAIEREK AEKFRKLQDA SRSAQALVEQ MVNEGVNADS     800
IKQASEQLNS RWIEFCQLLS ERLNWLEYQN NIIAFYNQLQ QLEQMTTTAE     850
NWLKIQPTTP SEPTAIKSQL KICKDEVNRL SGLQPQIERL KIQSIALKEK     900
GQGPMFLDAD FVAFTNHFKQ VFSDVQAREK ELQTIFDTLP PMRYQETMSA     950
IRTWVQQSET KLSIPQLSVT DYEIMEQRLG ELQALQSSLQ EQQSGLYYLS    1000
TTVKEMSKKA PSEISRKYQS EFEEIEGRWK KLSSQLVEHC QKLEEQMNKL    1050
RKIQNHIQTL KKWMAEVDVF LKEEWPALGD SEILKKQLKQ CRLLVSDIQT    1100
IQPSLNSVNE GGQKIKNEAE PEFASRLETE LKELNTQWDH MCQQVYARKE    1150
ALKGGLEKTV SLQKDLSEMH EWMTQAEEEY LERDFEYKTP DELQKAVEEM    1200
KRAKEEA              ·                                     1207
```

i.e. the 1st to 1207th of the sequence 1.

In the methods described above the peptide containing a part amino acid sequence encoded by the DMD gene may be a peptide containing a part of the amino acid sequence 2 shown by, for example:

a sequence of 10 to 200 amino acids from the sequence encoded by amino acids 1 to 1207 (inclusive);
a sequence of 10 to 200 amino acids from the sequence encoded by amino acids 1 to 1000 (inclusive);
a sequence of 10 to 200 amino acids from the sequence encoded by amino acids 1 to 600 (inclusive);
a sequence of 10 to 200 amino acids from the sequence encoded by amino acids 101 to 500 (inclusive);
a sequence of 10 to 200 amino acids from the sequence encoded by amino acids 101 to 400 (inclusive);
a sequence of 10 to 200 amino acids from the sequence encoded by amino acids 151 to 350 (inclusive);
a sequence of 10 to 100 amino acids from the sequence encoded by amino acids 1 to 1207 (inclusive);
a sequence of 10 to 100 amino acids from the sequence encoded by amino acids 1 to 1000 (inclusive);
a sequence of 10 to 100 amino acids from the sequence encoded by amino acids 1 to 600 (inclusive);
a sequence of 10 to 100 amino acids from the sequence encoded by amino acids 101 to 500 (inclusive);
a sequence of 10 to 100 amino acids from the sequence encoded by amino acids 101 to 400 (inclusive);
a sequence of 10 to 100 amino acids from the sequence encoded by amino acids 151 to 350 (inclusive);
a sequence of 10 to 100 amino acids from the sequence encoded by amino acids 191 to 290 (inclusive);
a sequence of 10 to 60 amino acids from the sequence encoded by amino acids 1 to 1207 (inclusive);
a sequence of 10 to 60 amino acids from the sequence encoded by amino acids 1 to 1000 (inclusive);
a sequence of 10 to 60 amino acids from the sequence encoded by amino acids 1 to 600 (inclusive);
a sequence of 10 to 60 amino acids from the sequence encoded by amino acids 101 to 500 (inclusive);
a sequence of 10 to 60 amino acids from the sequence encoded by amino acids 101 to 400 (inclusive);
a sequence of 10 to 60 amino acids from the sequence encoded by amino acids 151 to 350 (inclusive);
a sequence of 10 to 60 amino acids from the sequence encoded by amino acids 201 to 280 (inclusive);
a sequence of 10 to 60 amino acids from the sequence encoded by amino acids 211 to 270 (inclusive);
a sequence of 30 to 60 amino acids from the sequence encoded by amino acids 1 to 1207 (inclusive);
a sequence of 30 to 60 amino acids from the sequence encoded by amino acids 1 to 1000 (inclusive);
a sequence of 30 to 60 amino acids from the sequence encoded by amino acids 1 to 600 (inclusive);
a sequence of 30 to 60 amino acids from the sequence encoded by amino acids 101 to 500 (inclusive);
a sequence of 30 to 60 amino acids from the sequence encoded by amino acids 101 to 400 (inclusive);
a sequence of 30 to 60 amino acids from the sequence encoded by amino acids 151 to 350 (inclusive);
a sequence of 30 to 60 amino acids from the sequence encoded by amino acids 201 to 280 (inclusive);
a sequence of 30 to 60 amino acids from the sequence encoded by amino acids 211 to 270 (inclusive);
a sequence of 10 to 50 amino acids from the sequence encoded by amino acids 215 to 264 (inclusive);
a sequence of 20 to 50 amino acids from the sequence encoded by amino acids 215 to 264 (inclusive);

6

a sequence of 30 to 50 amino acids from the sequence encoded by amino acids 215 to 264 (inclusive);

a sequence of 40 to 50 amino acids from the sequence encoded by amino acids 215 to 264 (inclusive);

a sequence of 50 amino acids from the sequence encoded by amino acids 215 to 264 (inclusive);

a sequence of 10 to 200 amino acids from the sequence encoded by amino acids 201 to 600 (inclusive);

a sequence of 10 to 200 amino acids from the sequence encoded by amino acids 301 to 600 (inclusive);

a sequence of 10 to 200 amino acids from the sequence encoded by amino acids 391 to 590 (inclusive);

a sequence of 10 to 100 amino acids from the sequence encoded by amino acids 201 to 600 (inclusive);

a sequence of 10 to 100 amino acids from the sequence encoded by amino acids 301 to 600 (inclusive);

a sequence of 10 to 100 amino acids from the sequence encoded by amino acids 390 to 590 (inclusive);

a sequence of 10 to 100 amino acids from the sequence encoded by amino acids 416 to 515 (inclusive);

a sequence of 10 to 60 amino acids from the sequence encoded by amino acids 201 to 600 (inclusive);

a sequence of 10 to 60 amino acids from the sequence encoded by amino acids 301 to 600 (inclusive);

a sequence of 10 to 60 amino acids from the sequence encoded by amino acids 390 to 590 (inclusive);

a sequence of 10 to 60 amino acids from the sequence encoded by amino acids 425 to 504 (inclusive);

a sequence of 10 to 60 amino acids from the sequence encoded by amino acids 436 to 495 (inclusive);

a sequence of 30 to 60 amino acids from the sequence encoded by amino acids 201 to 600 (inclusive);

a sequence of 30 to 60 amino acids from the sequence encoded by amino acids 301 to 600 (inclusive);

a sequence of 30 to 60 amino acids from the sequence encoded by amino acids 390 to 590 (inclusive);

a sequence of 30 to 60 amino acids from the sequence encoded by amino acids 425 to 504 (inclusive);

a sequence of 30 to 60 amino acids from the sequence encoded by amino acids 436 to 495 (inclusive);

a sequence of 10 to 50 amino acids from the sequence encoded by amino acids 440 to 489 (inclusive);

a sequence of 20 to 50 amino acids from the sequence encoded by amino acids 440 to 489 (inclusive);

a sequence of 30 to 50 amino acids from the sequence encoded by amino acids 440 to 489 (inclusive);

a sequence of 40 to 50 amino acids from the sequence encoded by amino acids 440 to 489 (inclusive);

a sequence of 50 amino acids from the sequence encoded by amino acids 440 to 489 (inclusive);

The present invention also provides peptides comprising the aforesaid amino acid sequences of the methods.

The peptides described above may, for example, be peptide derivatives according to the following general formula:

X-A-R-A'-Y

wherein:

R represents a peptide described above;

A and A' are independently from 0 to 5 amino acid residues;

X represents a hydrogen atom or an alkyl group, aralkyl group, aryl group or acyl group which may be substituted or unsubstituted; and,

Y represents a hydroxy group or an unsubstituted amino group, or an alkylamino group, aralkylamino group or arylamino group which may be substituted or unsubstituted.

Suitably A and A' may independently comprise 0 to 3 amino acid residues.

Suitably A and A' may independently comprise 0 or 1 amino acid residues.

Where A and A' are each 0 or 1 they are preferably selected from Cys or Tyr.

Preferably X represents a hydrogen atom or a lower alkyl group having 1 to 6 carbon atoms, lower aralkyl group having 7 to 13 carbon atoms, aryl group having 6 to 12 carbon atoms or acyl group having 1 to 7 carbon atoms which may be substituted or unsubstituted; and, Y represents a hydroxy group or an unsubstituted amino group, or an alkylamino group having 1 to 6 carbon atoms, aralkylamino group having 7 to 13 carbon atoms or arylamino group having 6 to 12 carbon atoms which may be substituted or unsubstituted.

In the foregoing general formulae for the peptides, where X represents a hydrogen atom or an alkyl group having from 1 to 6 carbon atoms, an aralkyl group having from 7 to 13 carbon atoms, an aryl group having from 6 to 12 carbon atoms or an acyl group having from 1 to 7 carbon atoms, example substituents are fluorine atom, chlorine atom, bromine atom, iodine atom, oxygen atom, sulfur atom, nitrogen atom, phosphorous atom, a hydroxyl group, an alkoxy group having from 1 to 6 carbon atoms, an aralkyloxy group having from 7 to 13 carbon atoms, an aryloxy group having from 6 to 12 carbon atoms, an acyloxy group having from 1 to 6 carbon atoms, an amino group, a hydroxyamino group an alkylamino group having from 1 to 6 carbon atoms, an aralkylamino group having from 7 to 13 carbon atoms, an arylamino group having from 6 to 12 carbon atoms, an acylamino group having from 1 to 6 carbon atoms, a nitro group, a cyano group, an aminosulfonyl group, a carboxy group, an alkyloxycarbonyl group having from 2 to 8 carbon atoms, an aralkyloxycarbonyl group having from 8 to 14 carbon atoms, or an aryloxycarbonyl group having from 7 to 13 carbon atoms, and where Y represents a hydroxyl group, amino group, a hydroxyamino group, an alkylamino group having from 1 to 6 carbon atoms, an aralkylamino group having from 7 to 13 carbon atoms, an arylamino group having from 6 to 12 carbon atoms, an acylamino group having from 1 to 6 carbon atoms, example substituents are fluorine atom, chlorine atom, bromine atom, iodine atom, oxygen atom, sulfur atom, nitrogen atom, phosphorous atom, a hydroxyl group, an alkoxy group having from 1 to 6 carbon atoms, an aralkyloxy group having from 7 to 13 carbon atoms, an aryloxy group having from 6 to 12 carbon atoms, an acyloxy group having from 1 to 6 carbon atoms, an amino group, a hydroxyamino group, an alkylamino group having from 1 to 6 carbon atoms, an aralkylamino group having from 7 to 13 carbon atoms, an arylamino group having from 6 to 12 carbon atoms, an acylamino group having from 1 to 6 carbon atoms, a nitro group, a cyano group, an aminosulfonyl group, a carboxy group, an alkyloxycarbonyl group having from 2 to 8 carbon atoms, an aralkyloxycarbonyl group having from 8 to 14 carbon atoms, or an aryloxycarbonyl group having from 7 to 13 carbon atoms.

These peptides and derivatives thereof may be produced using the known chemical methods for peptide synthesis. Alternatively, they may be produced by biological methods using knowledge of the gene sequence coding for the peptides. For their use as antigens in the production of antiserum or antibodies, the peptides or derivatives thereof, may optionally be bound to high molecular weight carriers. Suitable carriers known in the art are bovine serum albumin, thyroglobulin, antitetanic toxoid, Keyhole limpet hemocyanin, etc. The peptide-carrier conjugate may be produced by conventional methods known in the art. The peptides or derivatives may also be used as antigens for the production of antiserum or antibodies without their being bound to high molecular weight carriers.

The mammals used for the production of antiserum according to the methods described above may be any of those mammals such as rabbit, goat, rat, mouse, horse, guinea pig, etc. commonly used for this purpose. The peptides or derivatives thereof may be administered to the mammals subcutaneously, intramuscularly, or intraperitoneally, either alone or with an adjuvant to enhance productivity of the antiserum or antibodies. For example, the adjuvant may be Freund's complete adjuvant, Freund's incomplete adjuvant. The antiserum and antibodies may be separated, purified and processed by techniques well known in the art.

The specimen sample tested by use of the antiserum or antibodies may, for example, be in the form of tissue sections, cell homogenates or body fluids such as blood or lymph. Tissue sections are suitably used for assay simplicity, or for cellular localization of the protein of interest.

The presence/absence of the protein of interest may be determined by use of the antibody in immunoassay techniques well known in the art and as briefly discussed below.

The antigen-antibody complex produced as a result of any binding between the protein and the antibody in the method described above, may be detected using techniques generally utilized in the immunochemical field, for example, by use of antibodies labelled with fluorescent, enzymic or radioactive moieties. Either a primary detection antibody (i.e. an antibody which is specific for the protein of interest) or a secondary detection antibody (i.e. an antibody which is specific for the primary detection antibody) may be labelled. Where the method uses a labelled secondary detection antibody the primary antibody will not be labelled.

As examples, fluorescein isothiocyanate may be used to detect a fluorescent label; peroxidase, alkaline phosphatase, or $\beta$-D-galactosidase, to detect an enzyme label. A suitable radio isotope label may be $^{125}I$ which is detected by virtue of its emissions, using for example, a scintillation counter. Labelling may be carried out by conjugation techniques well known in this field.

Any labelled antibody not bound in the antibody-antigen complex (either directly or via an unlabelled primary antibody) may be separated from labelled antibody bound in the complex by methods generally utilized in the art, for example: chromatography; electrophoresis; adsorption, and immobilised antibody etc. Which method is chosen will depend on factors such as the nature and properties of the specimen and the facilities available etc. Where the specimen sample is a tissue slice, any unbound label may be removed by washing and any immunoassay method for directly detecting binding of the label may be used.

As an alternative to directly detecting and measuring the binding of the label, the same information may also be indirectly determined by measuring the amount of unbound labelled antibody, which by comparison to the amount originally used in the test allows estimation of the amount which has been bound in the assay. Abbreviations and symbols used in the present specification have the following meanings.

1. Amino acid residues:
Ala, A : Alanine
Arg, R : Arginine
Asn, N : Asparagine
Asp, D : Aspartic acid
Cys, C : Cysteine
Gln, Q : Glutamine
Glu, E : Glutamic acid
Gly, G : Glycine
His, H : Histidine
Ile, I : Isoleucine
Leu, L : Leucine
Lys, K : Lysine
Met, M : Methionine
Phe, F : Phenylalanine
Pro, P : Proline
Ser, S : Serine
Thr, T : Threonine
Trp, W : Tryptophan
Tyr, Y : Tyrosine
Val, V : Valine

2. Protective groups:

Boc    tert-butyloxycarbonyl
Br Z    2-bromobenzyloxycarbonyl
Bzl    benzyl
Cl Z    2-chlcrobenzyloxycarbonyl
cHex    cyclohexyl
Dnp    2,4-dinitrophenyl
Tos    p-toluenesulfonyl

3. Reagents:

DCC    N,N'-dicyclohexylcarbodiimide
DIEA    N,N-diisopropylethylamine
DMF    N,N-dimethylformamide
EDT    1,2-ethanedithiol
HOBT    1-hydroxybenzotriazole
TFA    trifluoroacetic acid

4. Others:

M    molar concentration
N    normal concentration
Rt    retention time

The present invention will be described in more detail by referring to the examples below but it should not be considered as limited thereto. Reference is made to the figures in which:

Figure 1 shows localization of fluorescent labelled antibody in a tissue section taken from normal human muscle.

Figure 2 shows localization of fluorescent labelled antibody in a tissue section taken from the muscle of a patient suffering from Duchenne muscular dystrophy.

Example 1

Using the methods of solid phase peptide synthesis well known in the art, the peptide derivatives described in Example 2 and in the Examples subsequent thereto were synthesized.

1% Divinylbenzene-crosslinked styrene resin (Applied Biosystems Inc.) with introduced functional groups was used as an insoluble resin carrier.

All amino acids were in the L-form. For use in condensation reactions, the compounds had their amino groups and functional groups on the side chains protected as shown below:

Boc-Ala-OH
Boc-Arg(Tos)-OH
Boc-Asn-OH
Boc-Asp(OcHex)-OH
Boc-Gln-OH
Boc-Gly-OH
Boc-His(Dnp)-OH
Boc-His(Tos)-OH
Boc-Ile-OH
Boc-Leu-OH
Boc-Lys(ClZ)-OH
Boc-Met-OH
Boc-Phe-OH
Boc-Pro-OH
Boc-Ser(Bzl)-OH
Boc-Thr(Bzl)-OH
Boc-Trp(CHO)-OH
Boc-Tyr(BrZ)-OH
Boc-Val-OH

Extension of the peptide chain was carried out by sequentially condensing desired amino acids on the resin by the following procedures :

1. Treating with TFA-CH$_2$Cl$_2$ (1:1) for 90 seconds;
2. Treating with TFA-CH$_2$C$_{12}$ (6:4) for 14 minutes;
3. Washing with CH$_2$Cl$_2$ 3 times;
4. Washing twice with DIEA-DMF (1:9) for 45 seconds;
5. Washing with DMF 6 times;
6. Reacting with the DMF solution of activated amino acids prepared immediately before the reaction, for 15 to 30 minutes;
7. Washing with CH$_2$Cl$_2$ 6 times;
8. Checking the reaction state by the quantitative ninhydrin monitoring method (Virender K. Sarin, Stephen B H Kent, James P Tam and R.B. Merrifield, Anal. Biochem., 117, 147 (1981)] and repeating the procedures of Steps 3 to 7, if the reaction is insufficient; and

9. Repeating Steps 1 to 8 in accordance with the amino acid sequence.

However, where the amino acids Asn, Gln or Arg, were being sequentially condensed, Step 6 was modified as in the following step 6′:

6′. Reacting with the DMF solution of activated amino acids prepared immediately before the reaction for, 40 minutes to one hour and then washing with DMF twice.

Amino acid activation (excepting amino acids Asn, Gln and Arg) was carried out by the following procedures:

1. Dissolving 2 mmoles of the amino acid in 4 ml of $CH_2Cl_2$ (for Lys, Pro, His, Leu and Trp, however, a mixture of $CH_2Cl_2$-DMF was used);

2. Adding 2ml of a 0.5M solution of DCC in $CH_2Cl_2$ and then reacting for 5 minutes; and

3. Adding DMF while evaporating off the $CH_2Cl_2$ by flowing nitrogen gas into the solution, to form a solution of activated amino acid in DMF.

Activation of Asn, Gln and Arg was carried out by the following procedures:

1. Dissolving 2 mmoles of the amino acid in 4 ml of a 0.5 M solution of HOBT in DMF;

2. Adding 4 ml of a 0.5 M solution of DCC in $CH_2Cl_2$ and then reacting for 16 minutes; and

3. Adding DMF while evaporating off the $CH_2Cl_2$ by flowing nitrogen gas into the solution to form a solution of activated amino acid in DMF.

Example 2

Tyr-Glu-Lys-Gln-Ser-Asn-Leu-His-Arg-Val-Leu-Met-Asp-Leu-Gln-Asn-Gln-Lys-Leu-Lys-Glu-Leu-Asn-Asp-Trp-Leu-Thr-Lys-Thr-Glu-Glu-Arg-Thr-Arg-Lys-Met-Glu-Glu-Glu-Pro-Leu-Gly-Pro-Asp-Leu-Glu-Asp-Leu-Lys-Arg-Gln-NH₂.

p-methylbenzhydrylamine hydrochloride resin (0.5 mmoles) was obtained by introducing p-methylbenzhydrylamine onto the resin and this was then used as the starting material. Amino acids were condensed in accordance with the amino acid sequence to thereby extend the peptide chain and obtain 1.39 g of protected peptide-resin. His was protected as Boc-His(Tos)-OH. In order to examine condensation yield by the quantitative ninhydrin monitoring method, a part of the resin was withdrawn on the way.

p-Cresol (3 ml) and HF (20 ml) were added to the protected peptide-resin (1.16 g, 0.106 mmoles). After reacting at -2°C for an hour, HF was evaporated off in vacuo. Then, EDT (10 ml) and HF (10 ml) were added to the residue. After reacting at -2°C for an hour, HF was evaporated off in vacuo. Ether (40 ml) was added to the residue and the mixture was stirred. The supernatant was removed by decanting. This procedure was repeated twice and ether (40 ml) was further added to the residue and the mixture was stirred. Insoluble material was separated by filtration, washed with ether and dried to give colourless powders (1.09 g).

30 mls of an aqueous 8M urea solution was added to the obtained powder. After the insoluble material was filtered off, the system was fractionated by reversed phase high performance liquid chromatography (YMC AM-343 (20 mm x 250 mm) + GM340-5 (20 mm x 50 mm)), 0.1% TFA aqueous solution - $CH_3CN$ (25-31% linear gradient (96 minutes), 10 ml/min, poured separatedly in 5 portions). The fractions were analyzed by high performance liquid chromatography and the fractions containing the target compound were combined with each other. After $CH_3CN$ was evaporated off under reduced pressure, the combined solution was applied to reversed phase high performance liquid chromatography (YMC AM-343 (20 mm x 250 mm) + GM340-5 (20 mm x 50 mm)) for adsorption and retention of the target compound on the column. After washing the column with 0.5 N acetic acid, elution was performed with 0.5 N acetic acid-$CH_3CN$ (4:6). After $CH_3CN$ was evaporated off under reduced pressure, the residue was lyophilized to give a colourless fluffy powder (110 mg). Physical properties of the compound obtained:

Purity of peptide
98% (based on analysis data by HPLC)
Peptide content
86% (based on amino acid analysis of acid hydrolysate)
Reversed phase high performance liquid chromatography:
Rt = 25.9 min
(YMC AM-302 (4.6 mm x 150 mm), 0.1% TFA aqueous solution-0.1% TFA solution in $CH_3CN$ (0.50% linear gradient (30 minutes)), 1 ml/min
Amino acid analysis of hydrolysate:
Arg 4.00, Asp 6.30, Glu 11.59, Gly 1.00, His 1.03, Leu 8.62, Lys 5.78, Met 2.02, Pro 1.83, Ser 0.94, Thr 2.89, Tyr 1.09, Val 0.99, NH₃ 7.68
(6N-HCl, 100°C, 24 hours)
Mass Analysis m/z = 6267 (M + H)

Example 3

Pro-Glu-Asp-Val-Asp-Thr-Thr-Tyr-Pro-Asp-Lys-Lys-Ser-Ile-Leu-Met-Tyr-Ile-Thr-Ser-Leu-Phe-Gln-Val-Leu-Pro-Gln-Gln-Val-Ser-Ile-Glu-Ala-Ile-Gln-Glu-Val-Glu-Met-Leu-Pro-Arg-Pro-Pro-Lys-Val-Thr-Lys-Glu-Glu-NH₂

p-methylbenzhydrylamine hydrochloride resin (0.5 mmoles) was obtained by introducing p-methylbenzhydrylamine onto the resin and this was then used as the starting material. Amino acids were condensed in accordance with the amino acid sequence to thereby extend the peptide chain and obtain 3.73 g (76%) of protected peptide-resin.

Anisole (3 ml) and HF (20 ml) were added to the protected peptide-resin (1.56 g, 0.161 mmoles). After reacting at -2°C for an hour, HF was evaporated off in vacuo. Ether (40 ml) was added to the residue followed by stirring. The supernatant was removed by decanting. This procedure was repeated twice and ether (40 ml) was further added to the residue and the mixture was stirred. Insoluble matters were separated by filtration, washed with ether and dried to give pale yellow powders (1.22 g).

To the obtained powder 8 M urea aqueous solution was added. After insoluble matters were filtered off, 29% ammonium hydroxide was added to the system to make 2N ammonium hydroxide solution. The solution was stirred at 0°C for 30 minutes. TFA was added to render pH 4 and water was added to dilute to 2-fold volume. Urea was added to make an aqueous 8 M urea solution. Further TFA was added to adjust the pH to 2. The mixture was fractionated by reversed phase high performance liquid chromatography (YMC AM-343 (20 mm x 250 mm) + GM340-5 (20 mm x 50 mm), 0.1% TFA aqueous solution - CH₃CN (38-43% linear gradient (80 minutes)), 10 ml/min, poured separately in 6 portions). The fractions were analyzed by high performance liquid chromatography and the fractions containing the target compound were combined with each other. After CH₃CN was evaporated off under reduced pressure, the combined solution was applied to reversed phase high performance liquid chromatography (YMC AM-343 (20 mm x 250 mm) + GM340-5 (20 mm x 50 mm)) to adsorb and retain the target compound on the column. After washing the column with 0.5 N acetic acid, elution was performed with 0.5 N acetic acid-CH₃CN (4:6). After CH₃CN was distilled off under reduced pressure, the residue was lyophilized to give a colourless fluffy powder (158 mg).
Physical properties of the compound obtained:
Purity of peptide 99.6% (based on analysis date by HPLC)
Peptide content 83% (based on amino acid analysis of acid hydrolysate)
Reversed phase high performance liquid chromatography:
Rt = 7.4 min
(YMC AM-302 (4.6 mm x 150 mm), 0.1% TFA aqueous solution-0.1% TFA solution in CH₃CN (40-60% linear gradient (20 minutes)), 1 ml/min)
Amino acid analysis of hydrolysate:
Ala 1.00, Arg 0.95, Asp 2.90, Glu 9.75, Ile 3.40, Leu 3.42, Lys 3.96, Met 1.76, Phe 1.08, Pro 5.85, Ser 2.64, Thr 3.66, Tyr 1.74, Val 4.52, NH₃ 4.87
(6N-HCl, 110°C, 24 hours)
Mass analysis m/z = 5775 (M + H)

Example 4

Glu-Gly-Pro-Phe-Asp-Val-Gln-Glu-Thr-Glu-Ile-Ala-Val-Gln-Ala-Lys-Gln-Pro-Asp-Val-Glu-Glu-Ile-Leu-Ser-Lys-Gly-Gln-His-Leu-Tyr-Lys-Glu-Lys-Pro-Ala-Thr-Gln-Pro-Val-Lys-Arg-Lys-Leu-Glu-Asp-Leu-Ser-Ser-Glu-NH₂

p-methylbenzhydrylamine hydrochloride resin (0.2 mmoles) was obtained by introducing p-methylbenzhydrylamine onto the resin and this was then used as the starting material. Amino acids were condensed in accordance with the aminoacid sequence to thereby extend the peptide chain and obtain 1.54 g (89%) of protected peptide-resin. His was protected as Boc-His (Dnp)-OH.

5% Thiophenol solution in DMF (20 ml) was added to the protected peptide-resin (1.54 g, 0.156 mmoles). After stirring at room temperature for 30 minutes, the reaction mixture was washed with DMF 3 times. Again 5% thiophenol solution in DMF (20 ml) was added to the system. After stirring at room temperature for 30 minutes, the reaction mixture was washed 3 times with DMF and 3 times with DCM followed by drying. Anisole (3 ml) and HF (20 ml) were added to the system. After reaction at -2°C for an hour, HF was evaporated off in vacuo. Ether (40 ml) was added to the residue and the mixture was stirred. The supernatant was removed by decanting. This procedure was repeated twice and ether (40 ml) was further added to the residue and the mixture was stirred. Insoluble matters were taken out by filtration, washed with ether and dried to give pale yellow powders (1.01g).

To the obtained powders 8 M urea aqueous solution was added. After insoluble matters were filtered off, the system was fractionated by reversed phase high performance liquid chromatography (YMC AM-343 ( 20 mm x 50 mm) + GM340-5 ( 20 mm x 50 mm), 0.1% TFA aqueous solution - CH₃CN (24-29% linear gradient (80 minutes)), 10 ml/min, poured separately in 4 portions). The fractions were analyzed by high performance liquid chromatography and the fractions containing the target compound were combined with each other. After CH₃CN was evaporated off under reduced pressure, the combined solution was applied to reversed phase high performance liquid chromatography (YMC AM-343 ( 20 mm x 250 mm) + GM340-5 ( 20 mm x 50 mm)) to adsorb and retain the target compound on the column. After washing the column with 0.5 N acetic acid, elution was performed with 0.5 N acetic acid-CH₃CN (4:6). After CH₃CN was evaporated off under reduced pressure, the residue was lyophilized to give a colourless fluffy powder. (59.3 mg).
Physical properties of the compound obtained:
Purity of peptide
97.6% (based on analysis data by HPLC)
Peptide content
75% (based on amino acid analysis of acid hydrolysate)

Reversed phase high performance liquid chromatography:

Rt = 24.1 min

(YMC AM-302 ( 4.6 mm x 150 mm), 0.1% TFA aqueous solution-0.1% TFA solution in CH$_3$CN (0-50% linear gradient (30 minutes)), 1 ml/min)

Amino acid analysis of hydrolysate:

Ala 3.00, Arg 0.97, Asp 2.95, Glu 12.40, Gly 2.03, His 1.02, Ile 1.73, Leu 3.93, Lys 5.75, Phe 1.03, Pro 3.87, Ser 2.86, Thr 1.91, Tyr 0.94, Val 3.70, NH$_3$ 6.15

(6N-HCl, 110°C, 24 hours)

Mass analysis

m/z = 5664 (M + H)


Example 5

Leu-Ile-Ser-Leu-Glu-Ser-Glu-Glu-Arg-Gly-Glu-Leu-Glu-Arg-Ile-Leu-Ala-Asp-Leu-Glu-Glu-Glu-Asn-Arg-Asn-Leu-Gln-Ala-Glu-Tyr-Asp-Arg-Leu-Lys-Gln-Gln-His-Glu-His-Lys-Gly-Leu-Ser-Pro-Leu-Pro-Ser-Pro-Pro-Gln-NH$_2$

p-methylbenzhydrlylamine hydrochloride resin (0.204 mmoles) was obtained by introducing p-methylbenzhydrylamine onto the resin and this was then used as the starting material. Amino acids were condensed in accordance with the amino acid sequence to thereby extend the peptide chain and obtain 1.54g (73%) of protected peptide-resin. His was protected as Boc-His(Dnp)-OH.

5% Thiophenol solution in DMF (20 ml) was added to the protected peptide-resin (1.54 g, 0.149 mmoles). After stirring at room temperature for 30 minutes, the reaction mixture was washed with DMF 3 times. Again 5% thiophenol solution in DMF (20 ml) was added to the system. After stirring at room temperature for 30 minutes, the reaction mixture was washed 3 times with DMF and 3 times with DCM followed by drying. Anisole (3 ml) and HF (20 ml) were added to the system. After reaction at -2°C for an hour, HF was evaporated off in vacuo. Ether (40 ml) was added to the residue followed by stirring. The supernatant was removed by decanting. This procedure was repeated twice and ether (40 ml) was further added to the residue and the mixture was stirred. Insoluble matters were taken out by filtration, washed with ether and dried to give pale yellow powders (0.992g).

To the obtained powders 8 M urea aqueous solution (30 ml) was added. After insoluble matters were filtered off, the system was fractionated by reversed phase high performance liquid chromatography (YMC AM-343 (20 mm x 250 mm) + GM340-5 (20 mm x 50 mm), 0.1% TFA aqueous solution - CH$_3$CN (28-35% linear gradient (80 minutes)), 10 ml/min, poured separately in 6 portions). The fractions were analysed by high performance liquid chromatography and the fractions containing the target compound were combined with each other. After CH$_3$CN was evaporated off under reduced pressure, the combined solution was applied to reversed phase high performance liquid chromatography (YMC AM-343 (20 mm x 250 mm) + GM340-5 (20 mm x 50 mm)) to adsorb and retain on the column. After washing the column with 0.5 N acetic acid, elution was performed with 0.5 N acetic acid-CH$_3$CN (4:6). After CH$_3$CN was evaporated off under reduced pressure, the residue was lyophilized to give a colourless fluffy powder. (51.6 mg).

Physical properties of the compound obtained:

Purity of peptide

99.9% (based on analysis data by HPLC)

Peptide content

73% (based on amino acid analysis of acid hydrolysate)

Reversed phase high performance liquid chromatography:

Rt = 29.4 min

(YMC AM-302 (4.6 mm x 150 mm), 0.1% TFA aqueous solution-0.1% TFA solution in CH$_3$CN (0-50% linear gradient (30 minutes)), 1 ml/min)

Amino acid analysis of hydrolysate:

Ala 2.00, Arg 3.76, Asp 4.30, Glu 13.89, Gly 2.01, His 1.84, Ile 1.77, Leu 9.10, Lys 2.00, Pro 3.92, Ser 4.03, Tyr 0.87, NH$_3$ 5.90

(6N-HCl, 110°C, 24 hours)

Mass analysis

m/z = 5837 (M + H)


Example 6

Tyr-Glu-Arg-Glu-Asp-Val-Gln-Lys-Lys-Thr-Phe-Thr-Lys-Trp-Val-Asn-Ala-Gln-Phe-Ser-Lys-Phe-Gly-Lys-Gln-His-Ile-Glu-Asn-Leu-Phe-Ser-Asp-Leu-Gln-Asp-Gly-Arg-Arg-Leu-Leu-Asp-Leu-Leu-Glu-Gly-Leu-Thr-Gly-Gln-NH$_2$

p-methylbenzhydrylamine hydrochloride resin (0.284 mmoles) was obtained by introducing p-methylbenzhydrylamine onto the resin and this was then used as the starting material. Amino acids were condensed in accordance with the amino acid sequence to thereby extend the peptide chain and obtain 1.45 g (50%) of protected peptide-resin. His was protected as Boc-His(Dnp)-OH.

5% Thiophenol in DMF (20 ml) was added to the protected peptide-resin (1.45 g, 0.144 mmoles). After stirring at room temperature for 30 minutes, the reaction mixture was washed with DMF 3 times. Again 5% thiophenol in DMF (20 ml) was added to the system. After stirring at room temperature for 30 minutes, the

12

reaction mixture was washed 6 times with DMF and 3 times with DCM followed by drying. Anisole (3 ml) and HF (20 ml) were added to the system. After reacting at -2°C for an hour, HF was evaporated off in vacuo. Then, EDT (10 ml) and HF (10 ml) were added to the residue. After reacting at -2°C for an hour, HF was evaporated off in vacuo.

Ether (40 ml) was added to the residue and the mixture was stirred. The supernatant was removed by decanting. This procedure was repeated twice and ether (40 ml) was further added to the residue and the mixture was stirred. Insoluble matters were taken out by filtration, washed with ether and dried to give pale yellow powders (1.12 g).

To the obtained powders 1.0M mercaptoethanol in 6 M guanidine hydrochloride and 0.05M tris(hydroxymethyl)aminomethane solution (pH8.) was added, followed by stirring at room temperature for one hour.

After insoluble matters were separated by filtration, the system was fractionated by reversed phase high performance liquid chromatography (YMC AM-343 (20 mm x 250 mm) + GM340-5 (20 mm x 50 mm), 0.1% aqueous TFA-CH$_3$CN (33-39% linear gradient (140 minutes)), 10 ml/min, poured separately in 6 portions). The fractions were analyzed by high performance liquid chromatography and the fractions containing the target compound were combined with each other. After CH$_3$CN was evaporated off under reduced pressure, the combined solution was applied to reversed phase high performance liquid chromatography (YMC AM-343 (20 mm x 250 mm) + GM340-5 (20 mm x 50 mm)) to adsorb and retain the target compound on the column. After washing the column with 0.5 N acetic acid, elution was performed with 0.5 N acetic acid-CH$_3$CN (4:6). After CH$_3$CN was evaporated off under reduced pressure, the residue was lyophilized to give a colourless fluffy powder. (37.5 mg).

Physical properties of the compound obtained:

Purity of peptide 95.9% (based on analysis data by HPLC)

Peptide content 73% (based on amino acid analysis of acid hydrolysate)

Reversed phase high performance liquid chromatography:

Rt = 31.5 min

(YMC AM-302 (4.6 mm x 150 mm), 0.1% aqueous TFA -0.1% TFA in CH$_3$CN (0.50% linear gradient (30 minutes)), 1 ml/min)

Amino acid analysis of hydrolysate

Ala 1.00, Arg 2.57, Asp 5.69, Glu 8.55, Gly 3.96, His 0.85, Ile 0.87, Leu 6.78, Lys 4.66, Phe 3.93, Ser 1.80, Thr 2.65, Tyr 1.07, Val 1.80, NH$_3$ 8.72

(6N-HCl, 110°C, 24 hours)

Mass analysis

m/z = 5928 (M + H)

Example 7

2mg of the peptide Tyr-Glu-Lys-Gln-Ser-Asn-Leu-His-Arg-Val-Leu-Met-Asp-Leu-Gln-Asn-Gln-Lys-Leu-Lys-Glu-Leu-    Asn-Asp-Trp-Leu-Thr-Lys-Thr-Glu-Glu-Arg-Thr-Arg-Lys-Met-Glu-Glu-Glu-Pro-Leu-Gly-Pro-Asp-Leu-Glu-Asp-Leu-Lys-Arg-Gln-NH$_2$ in Freund's complete adjuvant was subcutaneously administered to rabbit (New Zealand white; female). This was followed by supplemental immunization performed by subcutaneous administration of the peptide twice every other two weeks together with the same amount of Freund's complete adjuvant.

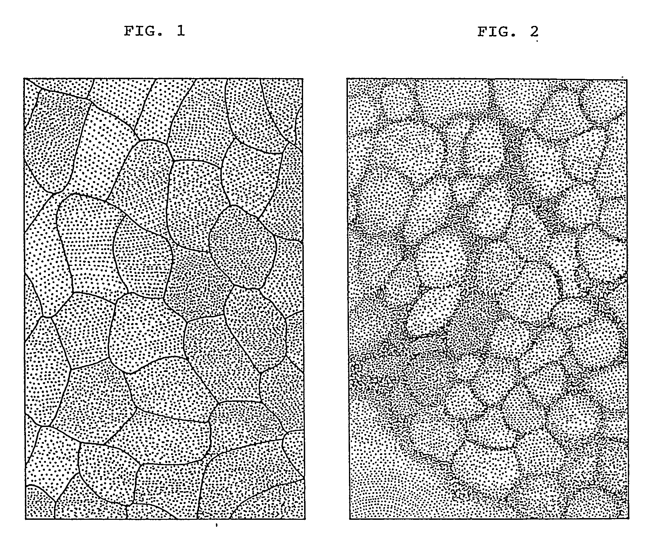

The antiserum obtained was diluted to 800-fold. A human muscle tissue section (thickness 4μm) was incubated with 30 μ1 of the antiserum dilution at 4°C for 10 hours. The section was then thoroughly washed with cold isotonic phosphate buffer saline (pH 7.4) and then incubated with 30 μl of fluorescein isothiocyanate labelled anti-rabbit IgG goat antibody (Tago Co.) at 20°C for 30 min. Washing was repeated. Fluorescence of the specimen was measured by use of a fluorescence microscope (Zeiss, Axiophot). Where the tissue section came from a normal human muscle, the fluorescence indicated that the antibody bound to protein on the cell membrane (Fig. 1). Where the tissue section was taken from the muscle of a patient suffering from Duchenne muscular dystrophy, no fluorescence was observed on the cell membrane (Fig. 2).

The foregoing results, show that the methods of the present invention allow the protein deletion or defect associated with Duchenne muscular dystrophy to be determined specifically in a simple manner.

Example 8

The peptide fragment was synthesized by the solid phase method on Applied Biosystems model 430A from position 215 to 264 of sequence 1 and 2 herein and purified with preparative reversed phase HPLC. The peptide consists of 50 amino acids, having a molecular weight of 5,775.

Balb/c mouse was immunized by a subcutaneous injection of 0.1 mg of the peptide mixed with Freund's complete adjuvant and two boosters of 0.1 mg of the peptide mixed in incomplete adjuvant with three-week interval. On the third day after the final booster, spleen cells were collected and fused with P3NP myeloma cell line under P4000 polyethylene glycol. Fifty-seven clones turned out to secrete an immunoglobulin(s). Six clones secreted antibody which stained the surface membrane of human quadriceps femoris muscle. One of the six clones, A1C, was recloned and the monoclonal antibody (McAb) was characterized by Western blot analysis and indirect immunofluorescence microscopy.

For Western blot analysis, about 50mg of muscle was minced finely by scissors in four volumes of the solution containing 70mM Tris-HCl (pH 6.7), 10% sodium dodecylsulfate, 10% ethylenediaminetetraacetic

13

acid, 5% 2-mercaptoethanol and 10% glycerol. It was boiled for three minutes, left at room temperature overnight and clarified by centrifugation at 16,000 x g for five minutes. Five µl of the supernatant was provided for SDS-PAGE. The methods of Doucet and Trifaro and Kyhse-Anderson were used for electrophoresis and electroblot, respectively. The proteins blotted on the nitrocellulose sheet were allowed to react with McAb AIC and the reaction was visualized with ABC kit (Vectastain, Vector Laboratories, Inc.).

For immunofluorescence microscopy, 8µm frozen sections of various muscle specimens were sequentially incubated with the antibody and FITC-labelled second antibody against mouse IgG. They were biopsied quadriceps femoris muscles from 19 patients with DMD, 8 patients with related neuromuscular diseases. The secions were observed under Zeiss epifluorescence microscope. For a study of chronological expression of dystrophin, biceps brachii muscles from legally-aborted fetuses were used.

Western blot analysis showed that McAb AIC reacted with a protein from normal human muscle extract migrated between laminin A and $\alpha$-spectrin. A minor band preceding the protein was observed and it might be a proteolytic fragment. The molecular weight of the protein was approximately 400 kd since the electrophoretic mobility was slightly larger than that of laminin A with the molecular weight of 450 kd. The antibody also bound to a high molecular weight protein of rabbit back muscle, which corresponds to the human 400 kd protein. The protein was not detected in muscle extract from a DMD patient. In addition, the McAb crossreacted with human $\alpha$-spectrin whereas no immunoreactivity was observed to other cytoskeletal proteins such as $\alpha$-actinin.

Intracellular distribution of the antigen in muscle fibres was characterized by indirect immunofluorescence microscopy. The McAb stained continuously the whole surface membrane of myofibres in cross section from human quadriceps femoris and rabbit hind limb muscles. Biopsied muscles of 8 related neuromuscular diseases, including 2 cases of polymyositis, each one case of facioscapulohumeral muscular dystrophy, limb-girdle dystrophy, rimmed vacuole myopathy, myotonic dystrophy, amyotrophic lateral sclerosis and floppy infant showed a clear and definite staining at the sarcolemma. No immunoreactive material was detected in peripheral nerves. Biceps brachii muscles from 17- and 35-week-old fetuses and 8-month-old floppy boy were also analysed for developmental expression of dystrophin. Some myofibres were partly stained in 17-week-old fetus while in 35-week-old fetus most of myofibres were stained uniformly at sarcolemma. In 8-month-old boy every muscle fibre, including type 1, 2A, 2B and 2C, was immunostained similarly to adult muscle.

In DMD, the immunoreactivity was hardly detected. Out of 19 DMD patients examined, myofibres from 3 patients were barely stained. A few myofibres from the other 16 patients were clearly stained, although a majority of fibres were not stained. The results demonstrate specificity and variety of defective expression of dystrophin in DMD.

## Claims

1. A method for assaying the protein deletion or defect associated with the human Duchenne muscular dystrophy gene, which comprises preparing a peptide which contains a part of an amino acid sequence encoded by the gene, or a derivative thereof, administering said peptide or derivative thereof to a mammal to form an antiserum, then combining the antiserum or an antibody fraction separated therefrom with a substance to be tested and assaying any antigen-antibody complex formed.

2. A method for assaying the protein deletion or defect associated with the human Duchenne muscular dystrophy gene, which comprises preparing a peptide which contains a part of an amino acid sequence encoded by the gene, or a derivative thereof, administering said peptide or a derivative thereof to a mammal, collecting spleen cells therefrom, making fused cells thereof, cloning said fused cells to form an antibody, then combining the antibody with a substance to be tested and assaying any antigen-antibody complex formed.

3. A method according to claim 1 or claim 2 wherein the amino acid sequence is:

```
MLWWEEVEDC YEREDVQKKT FTKWVNAQFS KFGKQHIENL FSDLQDGRRL      50
LDLLEGLTGQ KLPKEKGSTR VHALNNVNKA LRVLQNNNVD LVNIGSTDIV     100
DGNHKLTLGL IWNIILHWQV KNVMKNIMAG LQQTNSEKIL LSWVRQSTRN     150
YPQVNVINFT TSWSDGLALN ALIHSHRPDL FDWNSVVCQQ SATQRLEHAF     200
NIARYQLGIE KLLDPEDVDT TYPDKKSILM YITSLFQVLP QQVSIEAIQE     250
VEMLPRPPKV TKEEHFQLHH QMHYSQQITV SLAQGYERTS SPKPRFKSYA     300
YTQAAYVTTS DPTRSPFPSQ HLEAPEDKSF GSSLMESEVN LDRYQTALEE     350
VLSWLLSAED TLQAQGEISN DVEVVKDQFH THEGYMMDLT AHQGRYGNIL     400
QLGSKLIGTG KLSEDEETEV QEQMNLLNSR WECLRVASME KQSNLHRVLM     450
DLQNQKLKEL NDWLTKTEER TRKMEEEPLG PDLEDLKRQV QQHKVLQEDL     500
EQEQVRVNSL THMVVVYDES SGDHATAALE EQLKVLGDRW ANICRWTEDR     550
WVLLQDILLK WQRLTEEQCL FSAWLSEKED AVNKIHTTGF KDQNEMLSSL     600
QKLAVLKADL EKKKQSMGKL YSLKQDLLST LKNKSVTQKT EAWLDNFARC     650
WDNLVQKLEK STAQISQAVT TTQPSLTQTT VMETVTTVTT REQILVKHAQ     700
EELPPPPPQK KRQITVDSEI RKRLDVDITE LHSWITRSEA VLQSPEFAIF     750
RKEGNFSDLK EKVNAIEREK AEKFRKLQDA SRSAQALVEQ MVNEGVNADS     800
IKQASEQLNS RWIEFCQLLS ERLNWLEYQN NIIAFYNQLQ QLEQMTTTAE     850
NWLKIQPTTP SEPTAIKSQL KICKDEVNRL SGLQPQIERL KIQSIALKEK     900
GQGPMFLDAD FVAFTNHFKQ VFSDVQAREK ELQTIFDTLP PMRYQETMSA     950
IRTWVQQSET KLSIPQLSVT DYEIMEQRLG ELQALQSSLQ EQQSGLYYLS    1000
TTVKEMSKKA PSEISRKYQS EFEEIEGRWK KLSSQLVEHC QKLEEQMNKL    1050
RKIQNHIQTL KKWMAEVDVF LKEEWPALGD SEILKKQLKQ CRLLVSDIQT    1100
IQPSLNSVNE GGQKIKNEAE PEFASRLETE LKELNTQWDH MCQQVYARKE    1150
ALKGGLEKTV SLQKDLSEMH EWMTQAEEEY LERDFEYKTP DELQKAVEEM    1200
KRAKEEAQQK EAKVKLLTES VNSVIAQAPP VAQEALKKEL ETLTTNYQWL    1250
CTRLNGKCKT LEEYWACWHE LLSYLEKANK WLNEVEFKLK TTENIPGGAE    1300
EISEVLDSLE NLMRHSEDNP NQIRILAQTL TDGGYMDELI NEELETFNSR    1350
WRELHEEAVR RQKLLEQSIQ SAQETEKSLH LIQESLTFID KQLAAYIADK    1400

VDAAQMPQEA QKIQSDLTSH EISLEEMKKH NQGKEAAQRV LSQIDVAQKK    1450
LQDVSMKFRL FQKPANFELR LQESKMILDE VKMHLPALET KSVEQEVYQS    1500
QLNHCVNLYK SLSEVKSEVE MVIKTGRQIV QKKQTENPKE LDERVTALKL    1550
HYNELGAKVT ERKQQLEKCL KLSRKMRKEM NVLTEWLAAT DMELTKRSAV    1600
EGMPSNLDSE VAWGKATQKE IEKQKVHLKS ITEVGEALKT VLGKKETLVE    1650
DKLSLLNSNW IAVTSRAEEW LNLLLEYQKH METFDQNYDH ITKWIIQADT    1700
LLDESEKKKP QQKEDVLKRL KAELNDIRPK VDSTRDQAAN LMANRGDHCR    1750
KLVEPQISEL NHRFAAISHR IKTGKASIPL KELEQFNSDI QKLLEPLEAE    1800
IQQGVNLKEE DFNKDMNEDN EGTVKELLQR GDNLQQRITD ERKREEIKIK    1850
QQLLQTKHNA LKDLRSQRRK KALEISHQWY QYKRQADDLL KCLDDIEKKL    1900
ASLPEPRDER KIKEIDRELQ KKKEELNAVR RQAEGLSEDG AAMAVEPTQI    1950
QLSKRWREIE SKFAQFRRLN FAQIHTVREE TMMVMTEDMP LEISYVPSTY    2000
LTEITHVSQA LLEVEQLLNA PDLCAKDFED LFKQEESLKN IKDSLQQSSG    2050
RIDIIHSKKT AALQSATPVE RVKLQEALSQ LDFQWEKVNK MYKDRQGRFD    2100
RSVEKWRRFH YDIKIFNQWL TEAEQFLRKT QIPENWEHAK YKWYLKELQD    2150
GIGQRQTVVR TLNATGEEII QQSSKTDASI LQEKLGSLNL RWQEVCKQLS    2200
DRKKRLEEQK NILSEFQRDL NEFVLWLEEA DNIASIPLEP GKEQQLKEKL    2250
EQVKLLVEEL PLRQGILKQL NETGGPVLYS APISPEEQDK LENKLKQTNL    2300
QWIKVSRALP EKQGEIEAQI KDLGQLEKKL EDLEEQLNHL LLWLSPIRNQ    2350
LEIYNQPNQE GPFDVQETEI AVQAKQPDVE EILSKGQHLY KEKPATQPVK    2400
RKLEDLSSEW KAVNRLLQEL RAKQPDLAPG LTTIGASPTQ TVTLVTQPVV    2450
TKETAISKLE MPSSLMLEVP ALADFNRAWT ELTDWLSLLD QVIKSQRVMV    2500
GDLEDINEMI IKQKATMQDL EQRRPQLEEL ITAAQNLKNK TSNQEARTII    2550
TDRIERIQNQ WDEVQEHLQN RRQQLNEMLK DSTQWLEAKE EAEQVLGQAR    2600
AKLESWKEGP YTVDAIQKKI TETKQLAKDL RQWQTNVDYA NDLALKLLRD    2650
```

```
YSADDTRKVH  MITENINASW  RSIHKRVSER  EAALEETHRL  LQQFPLDLEK   2700
FLAWLTEAET  TANVLQDATR  KERLLEDSKG  VKELMKQWQD  LQGEIEAHTD   2750
YYHNLDENSQ  KILRSLEGSD  DAVLLQRRLD  NMNFKWSELR  KKSLNIRSHL   2800
EASSDQWKRL  HLSLQELLVW  LQLKDDELSR  QAPIGGDFPA  VQKQNDVHRA   2850
FKRELKTKEP  VIMSTLETVR  IFLTEQPLEG  LEKLYQEPRE  LPPEERAQNV   2900
TRLLRKQAEE  VNTEWEKLNL  HSADWQRKID  ETLERLQELQ  EATDELDLKL   2950
RQAEVIKGSW  QPVGDLLIDS  LQDHLEKVKA  LRGEIAPLKE  NVSHVNDLAR   3000
QLTTLGIQLS  PYNLSTLEDL  NTRWKLLQVA  VEDRVRQLHE  AHRDFGPASQ   3050
HFLSTSYQGP  WERAISPNKV  PYYINHETQT  TCWDHPKMTE  LYQSLADLNN   3100
VRFSAYRTAM  KLRRLQKALC  LDLLSLSAAC  DALDQHNLKQ  NDQPMDILQI   3150
INCLTTIYDR  LEQEHNNLVN  VPLCVDMCLN  WLLNVYDTGR  TGRIRVLSFK   3200
TGIISLCKAH  LEDKYRYLFK  QVASSTGFCD  QRRLGLLLHD  SIQIPRQLGE   3250
VASFGGSNIE  PSVRSCFQFA  NNKPEIEAAL  FLDWMRLEPQ  SMVWLPVLHR   3300
VAAAETAKHQ  AKCNICKECP  IIGFRYRSLK  HFNYDICQSC  FFSGRVAKGH   3350
KMHYPMVEYC  TPTTSGEDVR  DFAKVLKNKF  RTKRYFAKHP  RMGYLPVQTV   3400
LEGDNMETPV  TLINFWPVDS  APASSPQLSH  DDTHSRIEHY  ASRLAEMENS   3450
NGSYLNDSIS  PNESIDDEHL  LIQHYCQSLN  QDSPLSQPRS  PAQILISLES   3500
EERGELERIL  ADLEEENRNL  QAEYDRLKQQ  HEHKGLSPLP  SPPEMMPTSP   3550
QSPRDAELIA  EAKLLRQHKG  RLEARMQILE  DHNKQLESQL  HRLRQLLEQP   3600
QAEAKVNGTT  YSSPSTSLQR  SDSSQPMLLR  VVGSQTSDSM  GEEDLLSPPQ   3650
DTSTGLEEVM  EQLNNSFPSS  RGRNTPGKPM  REDTM
```

4. A method according to any one of claims 1, 2 or 3 wherein the peptide containing a part of the amino acid sequence is selected from:

a sequence of at least 10 amino acids from the sequence encoded by amino acids 1 to 3685 (inclusive);
a sequence of 10 to 200 amino acids from the sequence encoded by amino acid 1 to 3000 (inclusive);
a sequence of 10 to 200 amino acids from the sequence encoded by amino acids 1 to 2000 (inclusive);
a sequence of 10 to 200 amino acids from the sequence encoded by amino acids 1 to 1000 (inclusive);
a sequence of 10 to 200 amino acids from the sequence encoded by amino acids 1 to 500 (inclusive);
a sequence of 10 to 200 amino acids from the sequence encoded by amino acids 151 to 350 (inclusive);
a sequence of 10 to 100 amino acids from the sequence encoded by amino acids 1 to 3685 (inclusive);
a sequence of 10 to 100 amino acids from the sequence encoded by amino acids 1 to 3000 (inclusive);
a sequence of 10 to 100 amino acids from the sequence encoded by amino acids 1 to 2000 (inclusive);
a sequence of 10 to 100 amino acids from the sequence encoded by amino acids 1 to 1000 (inclusive);
a sequence of 10 to 100 amino acids from the sequence encoded by amino acids 1 to 500 (inclusive);
a sequence of 10 to 100 amino acids from the sequence encoded by amino acids 191 to 290 (inclusive);
a sequence of 10 to 60 amino acids from the sequence encoded by amino acids 1 to 3685 (inclusive);
a sequence of 10 to 60 amino acids from the sequence encoded by amino acids 1 to 3000 (inclusive);
a sequence of 10 to 60 amino acids from the sequence encoded by amino acids 1 to 2000 (inclusive);
a sequence of 10 to 60 amino acids from the sequence encoded by amino acids 1 to 1000 (inclusive);
a sequence of 10 to 60 amino acids from the sequence encoded by amino acids 1 to 500 (inclusive);
a sequence of 10 to 60 amino acids from the sequence encoded by amino acids 151 to 350 (inclusive);
a sequence of 10 to 60 amino acids from the sequence encoded by amino acids 190 to 290 (inclusive);
a sequence of 10 to 50 amino acids from the sequence encoded by amino acids 215 to 264 (inclusive);
a sequence of 20 to 50 amino acids from the sequence encoded by amino acids 215 to 264 (inclusive);
a sequence of 30 to 50 amino acids from the sequence encoded by amino acids 215 to 264 (inclusive);
a sequence of 40 to 50 amino acids from the sequence encoded by amino acids 215 to 264 (inclusive);
a sequence of 50 amino acids from the sequence encoded by amino acids 215 to 264 (inclusive);
a sequence of 10 to 200 amino acids from the sequence encoded by amino acids 201 to 600 (inclusive);
a sequence of 10 to 200 amino acids from the sequence encoded by amino acids 391 to 590 (inclusive);
a sequence of 10 to 100 amino acids from the sequence encoded by amino acids 201 to 600 (inclusive);
a sequence of 10 to 100 amino acids from the sequence encoded by amino acids 301 to 550 (inclusive);
a sequence of 10 to 100 amino acids from the sequence encoded by amino acids 416 to 515 (inclusive);
a sequence of 10 to 60 amino acids from the sequence encoded by amino acids 201 to 600 (inclusive);
a sequence of 10 to 60 amino acids from the sequence encoded by amino acids 301 to 550 (inclusive);
a sequence of 10 to 60 amino acids from the sequence encoded by amino acids 416 to 515 (inclusive);
a sequence of 10 to 60 amino acids from the sequence encoded by amino acids 436 to 495 (inclusive);
a sequence of 10 to 50 amino acids from the sequence encoded by amino acids 440 to 489 (inclusive);
a sequence of 20 to 50 amino acids from the sequence encoded by amino acids 440 to 489 (inclusive);

a sequence of 30 to 50 amino acids from the sequence encoded by amino acids 440 to 489 (inclusive);

a sequence of 40 to 50 amino acids from the sequence encoded by amino acids 440 to 489 (inclusive);

a sequence of 50 amino acids from the sequence encoded by amino acids 440 to 489 (inclusive);

a sequence of 10 to 200 amino acids from the sequence encoded by amino acids 1001 to 3000 (inclusive);

a sequence of 10 to 200 amino acids from the sequence encoded by amino acids 2001 to 3000 (inclusive);

a sequence of 10 to 200 amino acids from the sequence encoded by amino acids 2101 to 2600 (inclusive);

a sequence of 10 to 200 amino acids from the sequence encoded by amino acids 2286 to 2485 (inclusive);

a sequence of 10 to 100 amino acids from the sequence encoded by amino acids 1001 to 3000 (inclusive);

a sequence of 10 to 100 amino acids from the sequence encoded by amino acids 2001 to 3000 (inclusive);

a sequence of 10 to 100 amino acids from the sequence encoded by amino acids 2101 to 2600 (inclusive);

a sequence of 10 to 100 amino acids from the sequence encoded by amino acids 2335 to 2434 (inclusive);

a sequence of 10 to 60 amino acids from the sequence encoded by amino acids 1001 to 3000 (inclusive);

a sequence of 10 to 60 amino acids from the sequence encoded by amino acids 2001 to 3000 (inclusive);

a sequence of 10 to 60 amino acids from the sequence encoded by amino acids 2101 to 2600 (inclusive);

a sequence of 10 to 60 amino acids from the sequence encoded by amino acids 2356 to 2415 (inclusive);

a sequence of 10 to 50 amino acids from the sequence encoded by amino acids 2360 to 2409 (inclusive);

a sequence of 20 to 50 amino acids from the sequence encoded by amino acids 2360 to 2409 (inclusive);

a sequence of 30 to 50 amino acids from the sequence encoded by amino acids 2360 to 2409 (inclusive);

a sequence of 40 to 50 amino acids from the sequence encoded by amino acids 2360 to 2409 (inclusive);

a sequence of 50 amino acids from the sequence encoded by amino acids 2360 to 2409 (inclusive);

a sequence of 10 to 200 amino acids from the sequence encoded by amino acids 1001 to 3685 (inclusive);

a sequence of 10 to 200 amino acids from the sequence encoded by amino acids 2001 to 3685 (inclusive);

a sequence of 10 to 60 amino acids from the sequence encoded by amino acids 3470 to 3569 (inclusive);

a sequence of 10 to 50 amino acids from the sequence encoded by amino acids 3495 to 3544 (inclusive);

a sequence of 20 to 50 amino acids from the sequence encoded by amino acids 3495 to 3544 (inclusive);

a sequence of 30 to 50 amino acids from the sequence encoded by amino acids 3495 to 3544 (inclusive);

a sequence of 40 to 50 amino acids from the sequence encoded by amino acids 3495 to 3544 (inclusive);

a sequence of 50 amino acids from the sequence encoded by amino acids 3495 to 3544 (inclusive);

a sequence of 10 to 200 amino acids from the sequence encoded by amino acids 1 to 200 (inclusive);

a sequence of 10 to 100 amino acids from the sequence encoded by amino acids 1 to 200 (inclusive);

a sequence of 10 to 100 amino acids from the sequence encoded by amino acids 1 to 100 (inclusive);

a sequence of 10 to 60 amino acids from the sequence encoded by amino acids 1 to 100 (inclusive);

a sequence of 10 to 60 amino acids from the sequence encoded by amino acids 6 to 65 (inclusive);

a sequence of 10 to 50 amino acids from the sequence encoded by amino acids 11 to 60 (inclusive);

a sequence of 20 to 50 amino acids from the sequence encoded by amino acids 11 to 60 (inclusive);

a sequence of 30 to 50 amino acids from the sequence encoded by amino acids 11 to 60 (inclusive);

a sequence of 40 to 50 amino acids from the sequence encoded by amino acids 11 to 60 (inclusive); or

a sequence of 50 amino acids from the sequence encoded by amino acids 11 to 60 (inclusive);

in the aforesaid amino acid sequence 1.

5. A method according to claim 1 or claim 2 wherein the amino acid sequence is:

```
MLWWEEVEDC YEREDVQKKT FTKWVNAQFS KFGKQHIENL FSDLQDGRRL :        50
LDLLEGLTGQ KLPKEKGSTR VHALNNVNKA LRVLQNNNVD LVNIGSTDIV.       100
DGNHKLTLGL IWNIILHWQV·KNVMKNIMAG LQQTNSEKIL LSWVRQSTRN        150
YPQVNVINFT TSWSDGLALN ALIHSHRPDL FDWNSVVCQQ SATQRLEHAF        200
NIARYQLGIE KLLDPEDVDT TYPDKKSILM YITSLFQVLP QQVSIEAIQE        250
VEMLPRPPKV TKEEHFQLHH QMHYSQQITV SLAQGYERTS SPKPRFKSYA        300
YTQAAYVTTS DPTRSPFPSQ HLEAPEDKSF GSSLMESEVN LDRYQTALEE ·      350
VLSWLLSAED TLQAQGEISN DVEVVKDQFH THEGYMMDLT AHQGRVGNIL :      400
QLGSKLIGTG KLSEDEETEV QEQMNLLNSR WECLRVASME KQSNLHRVLM        450
DLQNQKLKEL NDWLTKTEER TRKMEEEPLG PDLEDLKRQV QQHKVLQEDL        500
EQEQVRVNSL THMVVVVDES SGDHATAALE EQLKVLGDRW ANICRWTEDR ·      550
WVLLQDILLK WQRLTEEQCL FSAWLSEKED AVNKIHTTGF·KDQNEMLSSL        600
QKLAVLKADL EKKKQSMGKL YSLKQDLLST LKNKSVTQKT·EAWLDNFARC·      650
WDNLVQKLEK STAQISQAVT·TTQPSLTQTT VMETYTTVTT REQILVKHAQ        700
EELPPPPPQK KRQITVDSEI RKRLDVDITE LHSWITRSEA VLQSPEFAIF        750
RKEGNFSDLK EKVNAIEREK AEKFRKLQDA SRSAQALVEQ MVNEGVNADS        800
IKQASEQLNS RWIEFCQLLS ERLNWLEYQN NIIAFYNQLQ QLEQMTTTAE        850
NWLKIQPTTP SEPTAIKSQL KICKDEVNRL SGLQPQIERL KIQSIALKEK ·      900
GQGPMFLDAD FVAFTNHFKQ VFSDVQAREK ELQTIFDTLP PMRYQETMSA        950·
IRTWVQQSET KLSIPQLSVT DYEIMEQRLG ELQALQSSLQ EQQSGLYYLS       1000
TTVKEMSKKA PSEISRKYQS EFEEIEGRWK KLSSQLVEHC QKLEEQMNKL       1050
RKIQNHIQTL KKWMAEVDVF LKEEWPALGD SEILKKQLKQ CRLLVSDIQT       1100
IQPSLNSVNE GGQKIKNEAE PEFASRLETE LKELNTQWDH MCQQVYARKE       1150
ALKGGLEKTV SLQKDLSEMH EWMTQAEEEY LERDFEYKTP DELQKAVEEM       1200
KRAKEEA                            .         .              1207
```

6. A method according to any one of claims 1, 2 or 5 wherein the peptide containing a part of the amino acid sequence is selected from:

a sequence of 10 to 200 amino acids from the sequence encoded by amino acids 1 to 1207 (inclusive);
a sequence of 10 to 200 amino acids from the sequence encoded by amino acids 1 to 1000 (inclusive);
a sequence of 10 to 200 amino acids from the sequence encoded by amino acids 1 to 600 (inclusive);
a sequence of 10 to 200 amino acids from the sequence encoded by amino acids 101 to 500 (inclusive);
a sequence of 10 to 200 amino acids from the sequence encoded by amino acids 101 to 400 (inclusive);
a sequence of 10 to 200 amino acids from the sequence encoded by amino acids 151 to 350 (inclusive);
a sequence of 10 to 100 amino acids from the sequence encoded by amino acids 1 to 1207 (inclusive);
a sequence of 10 to 100 amino acids from the sequence encoded by amino acids 1 to 1000 (in clusive);
a sequence of 10 to 100 amino acids from the sequence encoded by amino acids 1 to 600 (inclusive);
a sequence of 10 to 100 amino acids from the sequence encoded by amino acids 101 to 500 (inclusive);
a sequence of 10 to 100 amino acids from the sequence encoded by amino acids 101 to 400 (inclusive);
a sequence of 10 to 100 amino acids from the sequence encoded by amino acids 151 to 350 (inclusive);
a sequence of 10 to 100 amino acids from the sequence encoded by amino acids 191 to 290 (inclusive);
a sequence of 10 to 60 amino acids from the sequence encoded by amino acids 1 to 1207 (inclusive);
a sequence of 10 to 60 amino acids from the sequence encoded by amino acids 1 to 1000 (inclusive);
a sequence of 10 to 60 amino acids from the sequence encoded by amino acids 1 to 600 (inclusive);
a sequence of 10 to 60 amino acids from the sequence encoded by amino acids 101 to 500 (inclusive);
a sequence of 10 to 60 amino acids from the sequence encoded by amino acids 101 to 400 (inclusive);
a sequence of 10 to 60 amino acids from the sequence encoded by amino acids 151 to 350 (inclusive);
a sequence of 10 to 60 amino acids from the sequence encoded by amino acids 201 to 280 (inclusive);
a sequence of 10 to 60 amino acids from the sequence encoded by amino acids 211 to 270 (inclusive);
a sequence of 30 to 60 amino acids from the sequence encoded by amino acids 1 to 1207 (inclusive);
a sequence of 30 to 60 amino acids from the sequence encoded by amino acids 1 to 1000 (inclusive);
a sequence of 30 to 60 amino acids from the sequence encoded by amino acids 1 to 600 (inclusive);
a sequence of 30 to 60 amino acids from the sequence encoded by amino acids 101 to 500 (inclusive);
a sequence of 30 to 60 amino acids from the sequence encoded by amino acids 101 to 400 (inclusive);
a sequence of 30 to 60 amino acids from the sequence encoded by amino acids 151 to 350 (inclusive);
a sequence of 30 to 60 amino acids from the sequence encoded by amino acids 201 to 280 (inclusive);
a sequence of 30 to 60 amino acids from the sequence encoded by amino acids 211 to 270 (inclusive);
a sequence of 10 to 50 amino acids from the sequence encoded by amino acids 215 to 264 (inclusive);
a sequence of 20 to 50 amino acids from the sequence encoded by amino acids 215 to 264 (inclusive);

a sequence of 30 to 50 amino acids from the sequence encoded by amino acids 215 to 264 (inclusive);
a sequence of 40 to 50 amino acids from the sequence encoded by amino acids 215 to 264 (inclusive);
a sequence of 50 amino acids from the sequence encoded by amino acids 215 to 264 (inclusive);
a sequence of 10 to 200 amino acids from the sequence encoded by amino acids 201 to 600 (inclusive);
a sequence of 10 to 200 amino acids from the sequence encoded by amino acids 301 to 600 (inclusive);
a sequence of 10 to 200 amino acids from the sequence encoded by amino acids 391 to 590 (inclusive);
a sequence of 10 to 100 amino acids from the sequence encoded by amino acids 201 to 600 (inclusive);
a sequence of 10 to 100 amino acids from the sequence encoded by amino acids 301 to 600 (inclusive);
a sequence of 10 to 100 amino acids from the sequence encoded by amino acids 390 to 590 (inclusive);
a sequence of 10 to 100 amino acids from the sequence encoded by amino acids 416 to 515 (inclusive);
a sequence of 10 to 60 amino acids from the sequence encoded by amino acids 201 to 600 (inclusive);
a sequence of 10 to 60 amino acids from the sequence encoded by amino acids 301 to 600 (inclusive);
a sequence of 10 to 60 amino acids from the sequence encoded by amino acids 390 to 590 (inclusive);
a sequence of 10 to 60 amino acids from the sequence encoded by amino acids 425 to 504 (inclusive);
a sequence of 10 to 60 amino acids from the sequence encoded by amino acids 436 to 495 (inclusive);
a sequence of 30 to 60 amino acids from the sequence encoded by amino acids 201 to 600 (inclusive);
a sequence of 30 to 60 amino acids from the sequence encoded by amino acids 301 to 600 (inclusive);
a sequence of 30 to 60 amino acids from the sequence encoded by amino acids 390 to 590 (inclusive);
a sequence of 30 to 60 amino acids from the sequence encoded by amino acids 425 to 504 (inclusive);
a sequence of 30 to 60 amino acids from the sequence encoded by amino acids 436 to 495 (inclusive);
a sequence of 10 to 50 amino acids from the sequence encoded by amino acids 440 to 489 (inclusive);
a sequence of 20 to 50 amino acids from the sequence encoded by amino acids 440 to 489 (inclusive);
a sequence of 30 to 50 amino acids from the sequence encoded by amino acids 440 to 489 (inclusive);
a sequence of 40 to 50 amino acids from the sequence encoded by amino acids 440 to 489 (inclusive);
a sequence of 50 amino acids from the sequence encoded by amino acids 440 to 489 (inclusive);

7. A method according to claim 3 or claim 5 wherein the derivative of the peptide containing a part of the amino acid sequence is shown by the following general formulae:

X-A-R-A'-Y

wherein:

R represents any one of the peptides defined in claim 4 or claim 6;

A and A' are independently from 0 to 5 amino acid residues;

X represents a hydrogen atom or an alkyl group, aralkyl group, aryl group or acyl group which may be substituted o unsubstitited; and,

Y represents a hydroxy group or an unsubstituted amino group, or an alkylamino group, aralkylamino group or arlyamino group which may be substituted or unsubstituted.

8. A method according to claim 7 wherein A and A' may independently comprise 0 to 3 amino acid residues.

9. A method according to claim 7 or claim 8 wherein A and A' may independently comprise 0 or 1 amino acid residues.

10. A method according to any one of claims 7, 8 or 9 wherein A and A' are each 0 or 1 and selected from Cys or Tyr.

11. A method according to any one of claims 7, 8, 9 or 10 wherein preferably X represents a hydrogen atom or a lower alkyl group having 1 to 6 carbon atoms, lower aralkyl group having 7 to 13 carbon atoms, aryl group having 6 to 12 carbon atoms or acyl group having 1 to 7 carbon atoms which may be substituted or unsubstituted; and, Y represents a hydroxy group or an unsubstituted amino group, or an alkylamino group having 1 to 6 carbon atoms, aralkylamino group having 7 to 13 carbon atoms or arylamino group having 6 to 12 carbon atoms which may be substituted or unsubstituted.

12. A peptide containing part or all of the amino acid sequence defined in claim 4 or claim 6.

13. A peptide derivative defined in any one of claims 7, 8, 9, 10 or 11.

14. A method for making a specific binding member for the Duchenne muscular dystrophy protein which comprises administering the peptide or peptide derivative of any one of claims 12 or 13 to a mammal.

15. A reagent comprising a specific binding member for a peptide or derivative as defined in claim 12 or 13 and which detects the presence or absence of the Duchenne muscular dystrophy protein defect.

16. A kit for assaying the Duchenne muscular dystrophy protein defect which comprises a reagent according to claim 15.

FIG. 1

FIG. 2